# EUROPEAN PATENT APPLICATION

(11) **EP 2 392 297 A1**
(43) Date of publication of application: **07.12.2011**
(21) Application number: 09839139.4
(22) Date of filing: 21.12.2009
(51) Int. Cl.: A61F 5/455, A61F 5/44, A61F 5/453, A61F 13/15, A61F 13/49

(54) **BODILY FLUID TREATING ARTICLE AND WEARING ARTICLE INCLUDING SAME**

(30) Priority: 28.01.2009 JP 2009017328
(71) Applicant: Unicharm Corporation, Ehime 799-0111 (JP)
(72) Inventor: MITSUI, Koichiro, Kanonji-shi Kagawa 769-1602 (JP)
(74) Representative: Eke, Philippa Dianne
(86) International application number: PCT/JP2009/007044
(87) International publication number: WO 2010/086947

(57) **Abstract**

The present invention provides a bodily fluid treating article improved so as to assure that bodily fluids do not leak therefrom particularly when the wearer's body weight is exerted thereon or the wearer's posture changes, and a wearing article including the same. A bodily fluid treating article 1 is attached to a diaper 2 of a wearing article. A bodyside liner 61 and a backsheet 62 of the diaper 2 are formed with a chassis opening 59 extending therethrough in a thickness direction of them. A liquid-absorbent core 71 of a liquid-absorbent structure 70 is formed with a liquid-guiding opening 74 extending therethrough so as to be centrally aligned with the chassis opening 59. Within the liquid-guiding opening 74, a valve seat member 20 is provided as a component of the bodily fluid treating article 1 so that the outermost peripheral edge 25 of the valve seat member 20 may fit in the liquid-guiding opening 74. The opening 11 of the container 10 is exposed on the side facing the wearer's body by the intermediary of the liquid-guiding opening 74 and faces the wearer's external genitals.

## Description

### {Technical Field}

The present invention relates to bodily fluid treating articles and wearing articles including the same, and more particularly to disposable diapers, toilet-training pants, incontinent briefs and the like including bodily fluid treating articles.

### {Background}

Conventionally, it is well known, for example, from JP 2005-323797A (PTL 1) to form a bag-shaped absorbent article with an opening so that the wearer may discharge urine toward the opening. In this bodily fluid treating article of prior art, a guide sheet for dispersion of urine is interposed between the opening and a bottom of the bag-shaped absorbent article and opposite side edges of the guide sheet are covered with an absorbent structure serving to absorb discharged urine. A skin contact sheet is interposed between the guide sheet and the opening to prevent the discharged urine from coming in contact with the wearer's skin.

### {Citation List}

### {Patent Literature}

{PTL 1} JP 2005-323797A

### {Summary}

### {Technical Problem}

In the case of the above-mentioned absorbent article of prior art, urine once having been absorbed by the absorbent article partially leaches out from the absorbent structure, for example, under the wearer's body weight exerted thereon. With the absorbent structure from which urine is leaching out in this manner, there is a possibility that the urine once having been contained might move within the bag-shaped absorbent article and eventually leak out from the article through the opening if the wearer turns over on his or her side or gets down on all fours.

An object of the present invention is to provide a bodily fluid treating article improved so as to assure that bodily fluids do not leak from the bodily fluid treating article even when the wearer's body weight is exerted thereon or the wearer's posture changes and a wearing article including the same.

### {Solution to Problem}

The present invention includes a first aspect and a second aspect.
The present invention in the first aspect thereof relates to an improvement in a bodily fluid treating article having an opening through which bodily fluids flow into the article and including a container adapted to contain the bodily fluids flowing into the article.

The present invention in the first aspect thereof is **characterized in that** the bodily fluid treating article includes backflow preventing means adapted to restrict the possibility that bodily fluids once having been collected within the container might flow back from the container through the opening; the back flow preventing means includes a valve seat member located in the opening, a valve plug adapted to come in watertight contact with the valve seat member and a supporting member serving to prevent the valve plug from falling into the container wherein the bodily fluids can flow into the container when the valve seat member and the valve plug are spaced from each other.
The valve seat member and the supporting member may be formed of separately prepared members and then joined together or formed integrally of one and the same kind of material in the form of a single member.

The present invention in the first aspect thereof includes preferred embodiments as follows:
(1) The valve seat member includes a valve port extending from the interior to the exterior of the container; and the valve plug includes a small diameter segment having a diameter smaller than a diameter of the valve port, a contact region adapted to come in watertight contact with the valve port and a large diameter segment having a diameter larger than that of the valve port wherein the contact region is defined between the small diameter segment and the large diameter segment of the valve plug, and the small diameter segment is located on a side facing outward from the container and the large diameter segment is located on a side facing inward of the container.
(2) The valve plug has its density lower than a density of the bodily fluids.
(3) The supporting member has liquid-permeability and to be located on the side of valve plug facing inward of the container.

The present invention in the second aspect thereof relates to an improvement in a wearing article including a chassis which includes a longitudinal direction, a transverse direction, a side facing the wearer's body, a side facing away from the wearer's body, a front waist region, a rear waist region and a crotch region extending between the front and rear waist regions wherein the front waist region, the crotch region and the rear waist region are continuous in this order.
The present invention in the second aspect thereof is **characterized in that** the wearing article includes the bodily fluid treating article at least in the crotch region; and the opening of the bodily fluid treating article opens on the side facing the wearer's body.

The present invention in the second aspect thereof includes preferred embodiments as follows:
(1) The chassis includes a chassis opening formed in the crotch region and centrally aligned with the opening of the bodily fluid treating article, and the container lies on the side facing away from the wearer's body.
(2) The wearing article includes a liquid-absorbent structure at least in the crotch region; the liquid-absorbent structure includes a side facing the wearer's body, a side facing away from the wearer's body and a liquid-guiding opening extending through the liquid-absorbent structure; and the liquid-guiding opening is centrally aligned with the opening of the bodily fluid treating article and the container is located on the side opposite to the liquid-absorbent structure.

### {Advantageous Effects of Invention}

In the bodily fluid treating article according to the present invention in its first aspect, bodily fluids discharged by the wearer can reliably flow into the container and the bodily fluids once having been collected therein are prevented by the backflow preventing means from flowing back from the container. With this arrangement, leakage of bodily fluids from the container can be effectively prevented. The bodily fluid treating article includes a valve plug provided in the opening of the container so that this valve plug is adapted to come in watertight contact with the valve seat member. With this unique arrangement, leakage of bodily fluids from the container can be effectively restricted by the valve plug when coming in watertight contact with the valve seat member.

In the wearing article according to the present invention in its second aspect, the bodily fluid treating article is located at least in the crotch region so that the opening of the container opens toward the side facing the wearer's body. With this arrangement, when a diaper is used, for example, as the wearing article, urine discharged by the wearer can be contained in the bodily fluid treating article. In consequence, leakage of bodily fluids such as urine from the wearing article can be reliably restricted.

### {Brief Description of Drawings}

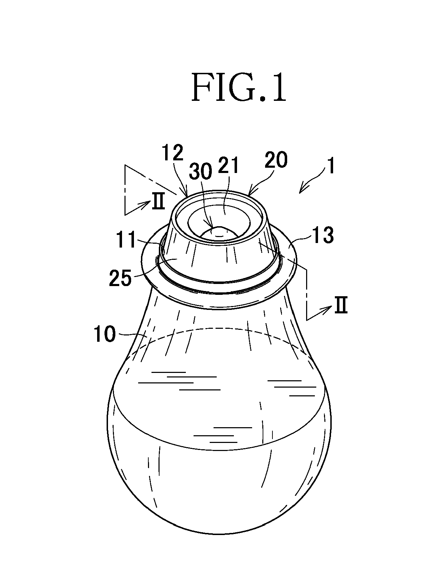

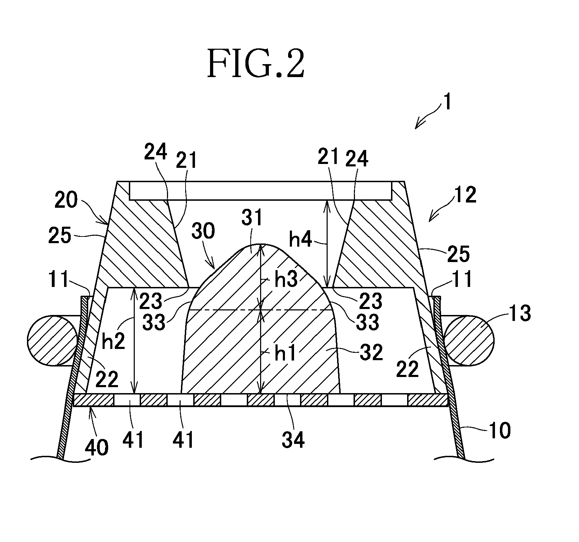

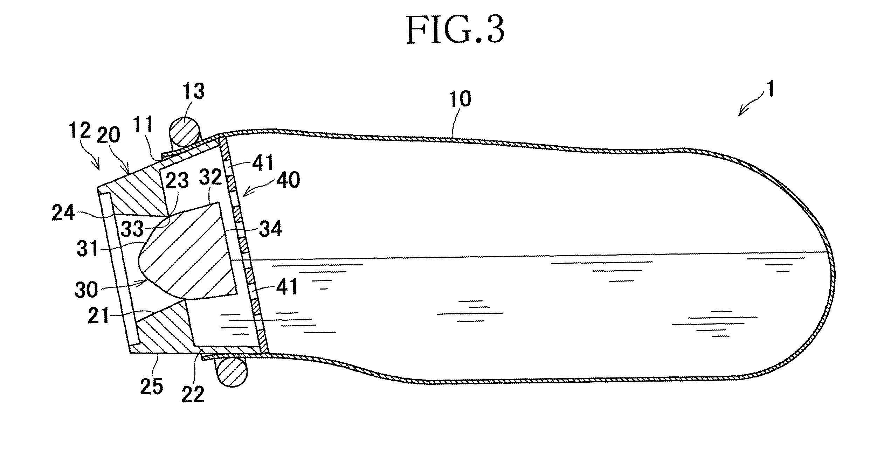

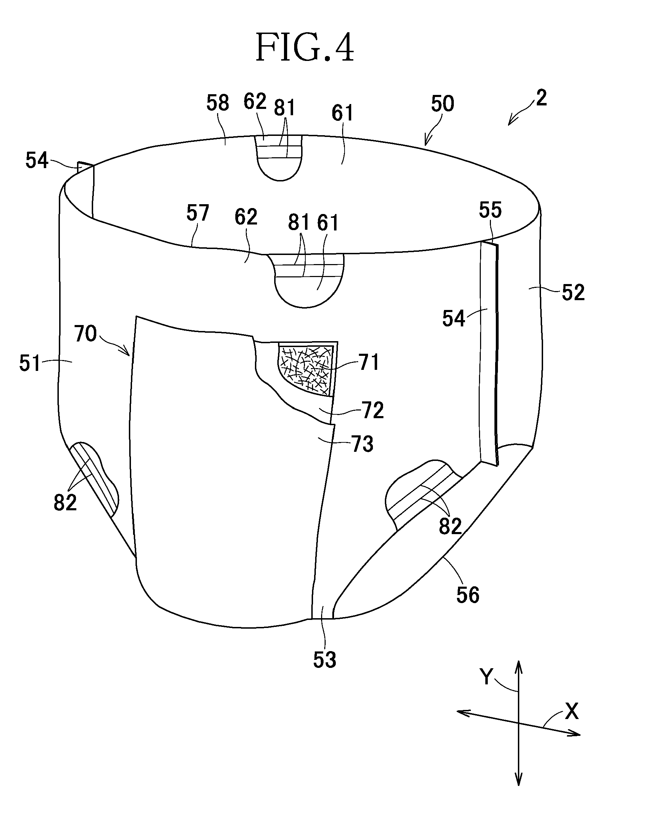

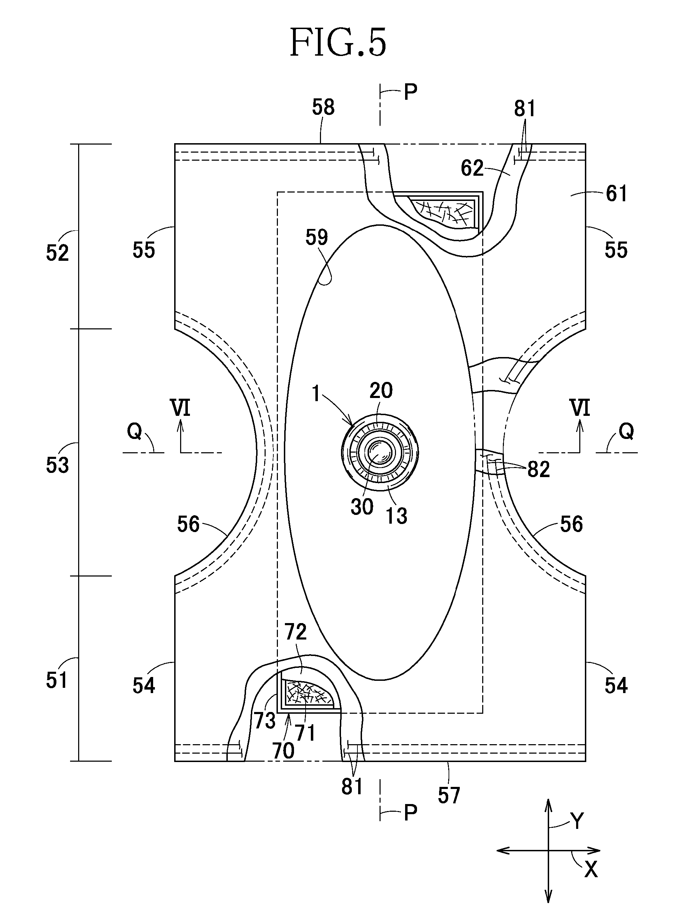

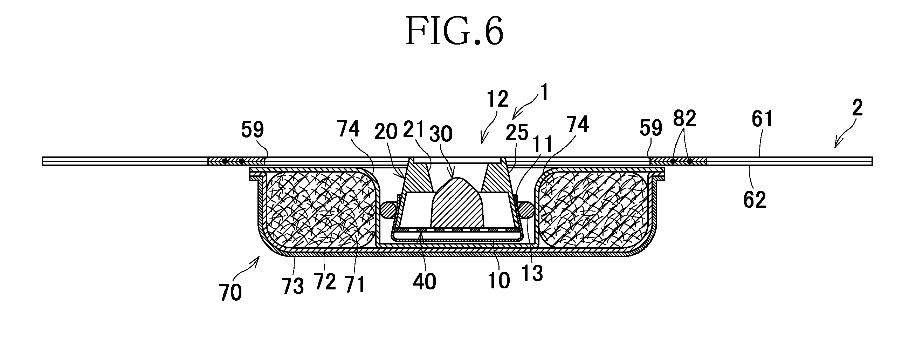

{Fig. 1} Perspective view of a bodily fluid treating article.
{Fig. 2} Schematic sectional view taken along line II-II in Fig. 1.
{Fig. 3} Diagram illustrating the bodily fluid treating article. {Fig. 4} Perspective view showing a diaper as put on the wearer's body.
{Fig. 5} Developed plan view of the diaper.
{Fig. 6 Schematic sectional view taken along line VI-VI in Fig. 5.

### {Description of Embodiments}

Figs. 1 and 2 exemplarily show the bodily fluid treating article 1 according to the present invention wherein Fig. 1 is the perspective view of the bodily fluid treating article 1 and Fig. 2 is the schematic sectional view taken along line II-II in Fig. 1. As will be apparent from Figs. 1 and 2, the bodily fluid treating article 1 includes a liquid-impervious container 10 formed with an opening 11 and a backflow preventing mechanism 12 arranged inside the opening 11.

The container 10 is bag-shaped and made of elastic resin such as natural rubber latex as main raw material. The container 10 made of such elastic resin that is extensible under the weight of bodily fluid to enlarge its available internal volume. Bodily fluids flow into the container 10 through the opening 11 formed therein.

The backflow preventing mechanism 12 includes an annular valve seat member 20 formed along the inner surface of the opening 11, a valve plug 30 formed inside the valve seat member 20 adapted to come in watertight contact with the innermost peripheral edge 23 of a valve port 21 defined by the valve seat member 20 and a supporting member 40 serving to prevent the valve plug 30 from falling off into the container 10. The valve port 21 includes the above-mentioned innermost peripheral edge 23 and the outermost peripheral edge 24 as viewed in a diametrical direction of the container 10. More specifically, the valve port 21 slopes up- and outward in the diametrical direction of the container 10 so that the valve port 21 has smallest diameter at the innermost peripheral edge 23 and largest diameter at the outermost peripheral edge 24. The valve plug 30 includes an outer peripheral surface 25 opposite to the inner peripheral surface of the valve port 21 and a peripheral wall extending from the outer peripheral surface into the container 10 defines a space forming segment 22. The space forming segment 22 forms a space in which the valve plug 30 is movably contained and has a diameter larger than those of the innermost peripheral edge 23 and the outermost peripheral edge 24. Such differential diameter results in the formation of a step between the space forming segment 22 and the innermost peripheral edge 23.

The valve seat member 20 is preferably formed, for example, of a resin having a sufficient stiffness to maintain an initial shape of the opening 11. The term "sufficient stiffness to maintain an initial shape of the opening 11" means the degree of stiffness assuring that at least the shape of the valve port 21 defined by the inner surface of the valve seat member 20 is maintained even when the wearer's body weight is exerted on the valve seat member 20. By maintaining the shape of the valve port 21, it is possible to restrict formation of a gap between the valve plug 30 and the innermost peripheral edge 23 of the valve seat member 20 when the valve plug 30 comes in contact with the innermost peripheral edge 23 of the valve seat member 20.
The outer peripheral surface 25 of the valve seat member 20 and the opening 11 of the container 10 are watertightly connected with each other by means of a connector member 13. The connector member 13 is formed of, for example, an O-ring made of elastic resin or the like and contractile force thereof functions to press the opening 11 against the outermost peripheral surface 25. It is also possible to connect the outermost peripheral surface 25 to the container 10 with adhesives or the like. In this case, the connector member is defined by adhesives.

The valve plug 30 is provided in the form of a column extending upward from the space forming segment 22 toward the valve port 21. The valve plug 30 includes a small diameter segment 31 having its diameter of the innermost peripheral edge 23, a large diameter segment 32 spaced downward from the small diameter segment 31 into the container 10 and a contact region 33 defined at a level between the small diameter segment 31 and the large diameter segment 32 and adapted to come in contact with the innermost peripheral edge 23. The contact region 33 is adapted to come in watertight contact with the innermost peripheral edge 23. To assure that the contact region 33 can come in watertight contact with the innermost peripheral edge 23 of the valve seat member 20, the valve plug 30 is preferably formed of a flexible resin such as polyurethane. The valve plug 30 includes a flat bottom segment 34 lying in the vicinity of a boundary between the backflow preventing mechanism 12 and the container 10.

The above-mentioned valve plug 30 is set to have a density lower than that of bodily fluids. In consequence, the valve plug 30 can float up from bodily fluids as bodily fluids are collected within the container 10. The valve plug 30 is supported by the supporting member 40 so as not to drop off into the container 10. The supporting member 40 lies on the side of the valve plug 30 facing the container 10, i.e., on the side of the space forming segment 22 of the valve seat member 20 facing the container 10. The supporting member 40 is liquid-pervious. Specifically, a resin plate is formed with a plurality of perforations 41 to obtain the liquid-pervious supporting member 40. Material for this supporting member 40 is not particularly specified as long as it is liquid-pervious and it may be appropriately selected from various kinds of materials. For example, liquid-pervious nonwoven fabric may be used as the material for the supporting member 40. In this way, it is assured that bodily fluids can permeate the supporting member 40 into the space forming segment 22.

A height dimension h1 of the valve plug 30 as measured from the contact region 33 to the bottom segment 34 is set to be smaller than the height h2 of the space forming segment 22 as measured from the innermost peripheral edge 23 to the supporting member 40. In consequence, assuming that the supporting member 40 is lowly-placed and the valve plug 30 is highly-placed as illustrated in Fig. 2, the contact region 33 of the valve plug 30 will be spaced from the innermost peripheral edge 23 of the valve seat member 20 and the bottom segment 34 of the valve plug 30 will be held in contact with the supporting member 40.

When the above-mentioned bodily fluid treating article 1 is in the state as illustrated in Figs. 1 and 2, bodily fluids such as urine discharged toward the valve port 21 flow through a gap defined between the peripheral surface of the small diameter segment 31 of the valve plug 30 and the innermost peripheral edge 23 and then through the perforations 41 of the supporting member 40 into the container since the valve plug 30 is spaced from the innermost peripheral edge 23 of the valve seat member 20. The container 10 is made of elastic resin and the weight of bodily fluids being collected gradually increase the volume of the container 10 and thereby prevents the inner volume of the container 10 from being filled with bodily fluids. The container 10 is adapted to be dynamically expanded in this manner, so that it is unnecessary to prepare the container 10 having a relatively large volume initially in production steps. Such feature advantageously contributes to make the bodily fluid treating article 1 compact.

When the bodily fluid treating article 1 has changed from its posture as illustrated in Figs. 1 and 2 to a posture lying in its side, bodily fluids move through the perforations 41 of the supporting member 40 to the bottom segment 34 of the valve plug 30 and this amount of bodily fluids forces the valve plug 30 to move outward from the side of the container 10. In addition, the density of the valve plug 30 is lower than that of bodily fluids and, in consequence, the valve plug 30 floats on bodily fluids. Floating of the valve plug 30 also serves to drive the valve plug 30 outward from the side of the container 10. The contact region 33 comes in contact with the innermost peripheral edge 23 as the valve plug 30 is driven outward from the side of the container 10. The valve plug 30 is sufficiently flexible to come in watertight contact with the innermost peripheral edge 23 and to prevent bodily fluids from leaking out of the container 10.
When the container 10 is placed upside down, the bottom segment 34 of the valve plug 30 is pressed by urine and the weight of the container 10 forces the contact region 33 to come in watertight contact with the innermost peripheral edge 23 to prevent bodily fluids from leaking out of the container 10.

In the above-mentioned bodily fluid treating article 1, a height dimension h3 as measured from a top of the small diameter segment 31 to the contact region 33 is preferably set to be smaller than a height dimension h4 as measured from the innermost peripheral edge 23 of the valve port 21 to the outermost peripheral edge 24. Such dimensional relationship assures that the top does not stick out beyond the outermost peripheral edge 24 when the contact region 33 of the valve plug 30 comes in contact with the innermost peripheral edge 23 of the valve seat member 20. Therefore, the top of the valve plug 30 should not come in contact with the wearer's skin and cause skin troubles. Even if the valve plug 30 comes in contact with the wearer's skin, an impact can be alleviated since the valve plug 30 is formed of flexible resin.

Figs. 4 through 6 exemplarily illustrate a wearing article including the bodily fluid treating article 1 as has been described above. Details of the wearing article will be described on the basis of a disposable diaper 2 as a typical example thereof.
Fig. 4 shows the diaper 2 put on the wearer's body and Fig. 5 shows the diaper 2 flatly developed after respective opposite side edges thereof have been peeled off one another wherein both Figs. 4 and 5 are partially cutaway for convenience of illustration. In Fig. 5, the diaper 2 is forcibly flattened against contractile force of respective elastics. Fig. 6 is a schematic sectional view taken along line VI-VI in Fig. 5. The diaper 2 includes a chassis 50, a liquid-absorbent structure 70, waist elastics 81 and leg elastics 82. The chassis 50 includes a front waist region 51, a rear waist region 52 and a crotch region 53 extending between the front and rear waist regions 51, 52 wherein the front waist region 51, the crotch region 53 and the rear waist region 52 are continuously arranged in this order. In addition, the chassis 50 has a longitudinal center line P-P bisecting a dimension of the diaper 2 in the transverse direction X and a transverse center line Q-Q bisecting a dimension of the diaper 2 in the longitudinal direction Y.

The chassis 50 includes a bodyside liner 61 defining its side facing the wearer's body and a backsheet 62 defining its side facing the wearer's garment wherein the above-mentioned waist elastics 81 and leg elastics 82 are sandwiched between these bodyside liner 61 and backsheet 62 and bonded to at least one of these bodyside liner 61 and backsheet 62. The chassis 50 includes front waist side edges 54 extending in the longitudinal direction Y, rear waist side edges 55 extending in the longitudinal direction Y and leg-opening rim 56 in the longitudinal direction Y in the crotch region 53.

The leg-opening rim 56 curve toward the longitudinal center line P-P in the crotch region 53 and the leg elastics 82 are attached under tension and in a contractible manner to the leg-opening rim 56 so as to extend therealong. The waist elastics 81 are attached under tension and in a contractible manner to the front and rear waist regions 51, 52 along front and rear ends 57, 58 of the chassis extending in the transverse direction X of the front and rear waist regions 51, 52. The front waist side edges 54 are joined to the rear waist side edges 55 so that the front and rear waist regions 51, 52 may become contiguous to each other and the diaper 2 may be pants-shaped.

The chassis 50 is formed at least in the crotch region 53 with a chassis opening 59 extending through the chassis 50 in its thickness direction. According to the present embodiment, the chassis opening 59 extends across the crotch region 53 into the front and rear waist regions 51, 52. The liquid-absorbent structure 70 lies on the side of the backsheet 62 facing the wearer's garment so as to close the chassis opening 59. The liquid-absorbent structure 70 has both sides facing the wearer's body and the wearer's garment, respectively, and includes a liquid-absorbent core 71 and a wrapping sheet 72 wherein the wrapping sheet 72 is provided on its side facing the wearer's garment with a liquid-impervious leakage-barrier sheet 73. The leakage-barrier sheet 73 may be additionally provided on its side facing the wearer's garment with a fibrous nonwoven fabric for improvement of texture. The bodyside liner 61 and the backsheet 62 may be formed, for example, of liquid-pervious fibrous nonwoven fabrics, the liquid-absorbent core 71 may be formed, for example, of a mixture of fluff wood pulp and super-absorbent polymer particles and the wrapping sheet 72 may be formed, for example, of a liquid-dispersant tissue paper. Otherwise, it is also possible to form these article components of the other types of materials widely used in the relevant technical field.

The liquid-absorbent core 71 of the liquid-absorbent structure 70 is formed with a liquid-guiding opening 74 extending through the core 71 in its thickness direction so that the liquid-guiding opening 74 may be centrally aligned with the chassis opening 59. This liquid-guiding opening 74 is provided with the valve seat member 20 constituting the bodily fluid treating article 1. More specifically, the valve seat member 20 is provided so that the outermost peripheral surface 25 of the valve seat member 20 may fit in the liquid-guiding opening 74 and the container 10 may be located between the liquid-absorbent structure 70 and the leakage-barrier sheet 73. The opening 11 of the container 10 is exposed on its side facing the wearer's body by the intermediary of the liquid-guiding opening 74 and faces the wearer's external genitals. In consequence, the wearer's external genitals face the opening 11 of the bodily fluid treating article 1 by the intermediary of the chassis opening 59 and the liquid-guiding opening 74.

A thickness dimension of the liquid-absorbent core 71 is larger than or equal to the thickness dimension of the bodily fluid treating article 1. The term "thickness dimension of the bodily fluid treating article" substantially means a length dimension as measured from the end of the valve seat member 20 facing the wearer's body to the supporting member 40. Such dimensional relationship makes it possible to restrict a possibility that the bodily fluid treating article 1 attached to the liquid-guiding opening 74 might stick out toward the wearer's body through the liquid-guiding opening 74. The opening 11 of the bodily fluid treating article 1 is preferably flush with the bodyside liner 61 and the backsheet 62 or lies closer to the wearer's garment than the bodyside liner 61 and backsheet 62.

Upon occurrence of urination onto the diaper 2 having been described above, urine flows into the container 10 of the bodily fluid treating article 1 through the opening 11. When the bodily fluid treating article 1 is in the posture as shown in Figs. 1 and 2, urine flows around the valve plug 30 and then into the container 10. When the posture of the bodily fluid treating article 1 changes from the upright posture to the posture as shown in Fig. 3 as the wearer has a lie-down or gets down on all fours, the valve plug 30 comes in watertight contact with the valve seat member 20 and thereby the urine once having been collected in the container 10 may be prevented from flowing back toward the side of the wearer's body. If the wearer's body weight is exerted on the bodily fluid treating article 1, for example, as the wearer gets seated, a level of urine certainly rises but the valve plug 30 having its density lower than that of bodily fluids can float on urine. Consequently, the valve plug 30 comes in contact with the valve seat member 20 before urine could flow out from the container 10. In this way, even in such case, it is possible to prevent urine from flowing back out of the bodily fluid treating article 1 and thereby urine leakage from the diaper 2 can be restricted.

Even if urination might occur besides the opening 11 of the bodily fluid treating article 1 or urine might leak out from the container 10, the liquid-absorbent structure 70 is present around the opening 11 and the urine which has leaked can be absorbed by this liquid-absorbent structure 70. In this manner, urine leakage from the diaper 1 can be further reliably restricted.
Only the opening 11 of the bodily fluid treating article 1 is exposed in the liquid-guiding opening 74 of the liquid-absorbent structure 70 and the remainder is held on the side of the wearer's garment so as not to stick out beyond the liquid-absorbent structure 70. With such unique arrangement, an uncomfortable feeling caused by contact of the bodily fluid treating article 1 with the wearer's body can be alleviated by the liquid-absorbent structure 70.

While the bodily fluid treating article 1 is formed with the single opening 11 in the present embodiment, it is possible to form the bodily fluid treating article 1 with two or more openings 11 and it is also possible to provide the wearing article with two or more bodily fluid treating articles 1.
While the liquid-absorbent structure 70 of the diaper 2 is formed with the liquid-guiding opening 74 in which the opening 11 of the bodily fluid treating article 1 is exposed in the present embodiment, it is also possible to locate the bodily fluid treating article 1 as a whole on the side of the liquid-absorbent structure 70 facing the wearer's body. In this case, the bodily fluid treating article 1 can be located between the chassis 50 and the liquid-absorbent structure 70.
While the chassis 50 is formed with the chassis opening 59 in the present embodiment, it is not essential to form the chassis opening 59. In that case, at least a region in which the opening 11 of the bodily fluid treating article is located may be made liquid-pervious to assure that bodily fluids such as urine can permeate the bodily fluid treating article 1 located on the side of the chassis 50 facing the wearer's garment. It is also possible to form the bodily fluid treating article 1 on the side of the chassis 50 facing the wearer's body. In either case, it is essential that bodily fluids such as urine can be reliably collected through the opening 11 of the bodily fluid treating article 1 into the container 10. Though a liquid-impervious container is used as the container 20 in the present embodiment, it is not limited thereto. As long as the container 10 is capable to contain bodily fluids flowing thereinto through the opening 11, it is possible to use a liquid pervious container, and in such a case, the liquid-absorbent core may be used to contain bodily fluids once collected in such a liquid pervious container.

While the valve seat member 20 and the supporting member 40 are formed of the members separately prepared and then joined together in the present embodiment, it is also possible to form these two members 20, 40 integrally. When the valve seat member 20 and the supporting member 40 are integrally formed, it is also possible to form both members by one and the same kind of material in the form of a single member.

### {Reference Signs List}

- 1: bodily fluid treating article
- 2: diaper (wearing article)
- 10: container
- 11: chassis opening
- 12: backflow preventing mechanism
- 20: valve seat member
- 21: valve port
- 30: valve plug
- 31: small diameter segment
- 32: large diameter segment
- 33: contact region
- 40: supporting member
- 50: chassis
- 51: front waist region
- 52: rear waist region
- 53: crotch region
- 59: chassis opening
- 70: liquid-absorbent structure
- 74: liquid-guiding opening

## Claims

1. A bodily fluid treating article having an opening through which bodily fluids flow into the article and an absorbent structure adapted to contain the bodily fluids flowing into the article, the bodily fluid treating article being **characterized in that**:
the bodily fluid treating article includes backflow preventing means adapted to restrict a possibility that the bodily fluids once having been collected within the container might flow back from the container through the opening;
the backflow preventing means comprises a valve seat member located in the opening, a valve plug adapted to come in watertight contact with the valve seat member and a supporting member serving to prevent the valve plug from falling into the container wherein the bodily fluids can flow into the container when the valve seat member and the valve plug are spaced from each other.

2. The bodily fluid treating article defined by claim 1, wherein:
the valve seat member includes a valve port extending from the interior to the exterior of the container;
and the valve plug comprises a small diameter segment having a diameter smaller than a diameter of the valve port, a contact region adapted to come in watertight contact with the valve port and a large diameter segment having a diameter larger than the diameter of the valve port wherein the contact region is defined between the small diameter segment and the large diameter segment of the valve plug, the small diameter segment defined at a level of the valve plug more remote than the large diameter segment outward from the container.

3. The bodily fluid treating article defined by claim 1 or 2, wherein the valve plug is set to have its density lower than a density of the bodily fluids.

4. The bodily fluid treating article defined by any one of claims 1 through 3, wherein the supporting member is liquid pervious and located on the side of the valve plug facing the container.

5. A wearing article including a chassis which comprises a longitudinal direction, a transverse direction, a side facing the wearer's body, a side facing away from the wearer's body, a front waist region, a rear waist region and a crotch region extending between the front and rear waist regions wherein the front waist region, the crotch region and the rear waist region are continuous in this order, the wearing article being **characterized in that**:
the wearing article includes the bodily fluid treating article defined by claim 1 at least in the crotch region; and
the opening of the bodily fluid treating article opens on the side facing the wearer's body.

6. The wearing article defined by claim 5, wherein the chassis includes a chassis opening formed in the crotch region and centrally aligned with the opening of the bodily fluid treating article and the container lies on the side facing away from the wearer's body.

7. The wearing article defined by claim 5 or 6, wherein:
the wearing article includes a liquid-absorbent structure at least in the crotch region;
the liquid-guiding opening is centrally aligned with the opening of the bodily fluid treating article and the container is located on the side opposite to the liquid-absorbent structure.
